# EUROPEAN PATENT SPECIFICATION

(11) **EP 1 048 650 B1**
(45) Date of publication and mention of the grant of the patent: **26.02.2003**
(21) Application number: 00303633.2
(22) Date of filing: 28.04.2000
(51) Int. Cl.: C07D 209/52

(54) **Process for producing 2-azabicyclo (2.2.1) hept-5-en-3-one**
Verfahren zur Herstellung von 2-Azabicyclo[2.2.1]hept-5-en-3-on
Procédé pour la fabrication de 2-azabicyclo[2.2.1]hept-5-en-3-one

(30) Priority: 30.04.1999 JP 12401599
(43) Date of publication of application: 02.11.2000
(73) Proprietor: KURARAY CO., LTD., Kurashiki-City Okayama Prefecture 710 (JP)
(72) Inventor: Fukumoto, Takashi, c/o Kuraray Co., Ltd., Kitakanbara-gun, Niigata-Pref. (JP); Ikarashi, Rensuke, c/o Kuraray Co., Ltd., Kitakanbara-gun, Niigata-Pref. (JP)
(74) Representative: Nachshen, Neil Jacob

(56) References cited:
- EP-A- 0 926 136
- JP-A- 8 027 110
- CHEMICAL ABSTRACTS, vol. 121, no. 9, 29 August 1994 (1994-08-29) Columbus, Ohio, US; abstract no. 108525f, HAYASHIBARA, HIDEO ET AL.: "Preparation of 2-azabicyclo(2.2.1)hept-5-en-3-one." XP002146535 & JP 5 331 139 A (KURARAY CO) 24 December 1993
- CHEMICAL ABSTRACTS, vol. 127, no. 3, 21 July 1997 (1997-07-21) Columbus, Ohio, US; abstract no. 34139h, "Process for the preparation of 2-azabicyclo(2.2.1)hept-5-en-3-one by ccloaddition." XP002146536 & JP 9 165 372 A (KURARAY CO.) 24 June 1997

## Description

### Field of the Invention

The present invention relates to a process for producing 2-azabicyclo[2.2.1]hept-5-en-3-one (which may be abbreviated to ABH hereinafter), which is an intermediate for synthesizing carbocyclic nucleosides that are useful for medicines such as an anti-virus agent.

Since carbocyclic nucleosides have a structure in which an oxygen atom constituting a furanose ring of the nucleoside is substituted with a methylene group and the structure is very similar to a natural nucleoside having a furanose ring, it can act as a substrate or an inhibitor for various enzymes in living bodies. Further, since the carbocyclic nucleoside has no glycoside bonding, they are not subjected to cleaving or splitting by enzymes such as nucleoside phospholylase or nucleoside hydrase. Since they have different metabolic pathways from natural nucleosides having the furanose ring, they exhibit various physiological activities. For example, carbocyclic adenosine known as Aristeromycin is a carbocyclic nucleoside, which is a metabolite of Streptomyces citricolor and has been noted in view of its strong cytotoxicity as being different from nucleosides having the furanose ring.

Further, carbocyclic-2,3-dideoxy-2,3-didehydroguanosine, is carbocyclic nucleoside that has now been developed as an anti-HIV agent [R. Vince et al., Biochem, Biophys. Res. Commun. 156, 1046 (1988)].

ABH is a compound that is most frequently used as an intermediate for pure-chemically synthesizing a carbocyclic moiety of these carbocyclic nucleosides, such as 2 α,3α-dihydroxy-4β-aminocyclopentanone-1β-methanol or cis-4-aminocyclopent-2-en-1β -methanol [R. Vince, et al., J. Org. Chem., **43**, 2311 (1978); B. L. Kamm et al., J. Org. Chem., **46**, 3268 (1981); W. C. Faith et al., J. Org. Chem., **50**, 1983 (1985)].

### Description of the Related Art

As a method for synthesizing ABH, there has been known a process of subjecting cyclopentadiene and p-toluenesulfonyl cyanide to cycloaddition reaction to form 3-p-toluenesulfonyl-2-azabicyclo[2.2.1]hepta-2,5-diene as an intermediate and removing the toluenesulfonyl group on the 3-position in the intermediate by using acetic acid [J. C. Jagt et al., J. Org. Chem., **39**, 564 (1974); R. Vince et al., J. Org. Chem., **43**, 2311 (1978)].

However, the synthesizing process described above involves various problems, for example, in that ① cyclopentadiene which may be used theoretically in an equimolar amount to p-toluenesulfonyl cyanide has to be used actually in a great excess of 15 to 35 molar times inclusive; ② 3-p-toluenesulfonyl-2-azabicyclo[2.2.1]hepta-2,5-diene obtained by the reaction of p-toluenesulfonyl cyanide and cyclopentadiene has to be condensed and taken out as lumps, which are pulverized into powder and then reacted with acetic acid; 3 acetic acid has to be added in a great excess of 5 to 23 molar times all at once when removing the toluenesulfonyl group at the 3-position by treating 3-p-toluenesulfonyl-2-azabicyclo[2.2.1]hepta-2,5-diene with acetic acid, meaning that an abrupt exothermic reaction has to be controlled; ④ if the exothermic reaction in the ③ cannot be controlled satisfactorily and the reaction temperature rises excessively, the aimed product, ABH, cannot be obtained at all or is obtainable only at an extremely low yield, ⑤ solid by-products are formed upon the reaction in step ③, which hinders smooth stirring and the reaction cannot be proceeded smoothly; and ⑥ a great amount of waste water is formed increasing the burden for treating the same. Therefore, by the synthesis process described above, ABH cannot be produced industrially satisfactory in view of economy and safety.

It has previously been observed that it is possible to produce ABH in a high purity and a high yield with safety and a good productivity by reacting sulfonyl cyanide such as p-toluenesulfonyl cyanide with cyclopentadiene under a condition of a reduced amount of the reagent and solvent. Thus, the present inventors have previously developed the following processes;
(i) a process for producing ABH by way of a first step of condensing sulfonyl cyanide and cyclopentadiene in a hydrocarbon solvent and then a second step of treating with water (Japanese Published Unexamined Patent Application Hei 5-331139),
(ii) a process for producing ABH by reacting sulfonyl cyanide and cyclopentadiene in water or a mixed solvent of water and a hydrocarbon (Japanese Published Unexamined Patent Application Hei 5-331140),
(iii) a process for producing ABH by reacting benzenesulfonyl cyanide and cyclopentadiene in a mixed solvent of water and a water soluble solvent under a pH condition of from 3 to 4 inclusive (Japanese Published Unexamined Patent Application Hei 8-27110); and
(iv) a process for producing ABH by reacting sulfonyl cyanide and cyclopentadiene in a hydrocarbon solvent to form 3-sulfonyl-2-azabicyclo[2.2.1]hepta-2,5-diene as an intermediate product and hydrolyzing the intermediate product by adding a solution of the intermediate product into a mixed solvent of water and a water soluble solvent under a pH condition of from 3 to 7 inclusive (Japanese Published Unexamined Patent Application Hei 9-165372).

When compared with the existing process by J. C. Jagt et al. [namely, a process for producing ABH by reacting acetic acid with 3-p-toluenesulfonyl-2-azabicyclo[2.2.1]hepta-2,5-diene prepared by the reaction of cyclopentadiene and p-toluenesulfonyl cyanide], the production processes (i)-(iv) described above have various advantages such that:
(a) it is not necessary to use cyclopentadiene in a great excess relative to sulfonyl cyanide;
(b) they require no troublesome procedures of taking out 3-p-toluenesulfonyl-2-azabicyclo[2.2.1]hepta-2,5-diene formed as the intermediate product in a condensed form, pulverizing the same into powder, and then subjecting the powder to the succeeding step;
(c) since no abrupt exothermic reaction takes places, reaction control is easy and it is excellent in safety;
(d) the yield of the aimed ABH is high;
(e) solid by-products which hinder the stirring during the reaction are less formed comparatively; and
(f) the amount of waste water to be treated is small to moderate the processing burden.

Each of the processes (i) and (iv) above is conducted by way of two steps of reacting sulfonyl cyanide and cyclopentadiene in a hydrocarbon solvent in the first step to form 3-sulfonyl-2-azabicyclo[2.2.1]hepta-2,5-diene as an intermediate product and then processing the solution of the intermediate product in water in the process (i) or a mixed solvent of water and a water soluble solvent in the process (iv) in the second step to produce ABH.

In these processes, however, it is necessary to handle 3-sulfonyl-2-azabicyclo[2.2.1]hepta-2,5-diene, which is relatively unstable. In view of this point, there remains a room for improvement.

On the other hand, each of the processes (ii) and (iii) above directly produce ABH by reacting sulfonyl cyanide and cyclopentadiene in water or a mixed solvent of water and a hydrocarbon solvent or in a mixed solvent of water and a water soluble solvent in one step. These processes are more simple than the two step processes (i) or (iv) above and can be said to be industrially advantageous.

However, in the method (ii), the pH is not controlled during the reaction of sulfonyl cyanide and cyclopentadiene, and it has been found that the pH in the reaction mixture is 3 or lower (generally pH 2 to 3 inclusive) due to sulfonyl cyanide present in the mixture or sulfinic acid (for example, benzenesulfinic acid) formed by reaction. In this case, it is difficult to completely prevent deposition, in the reaction mixture, of solid products such as dimerization products (for example, benzenesulfinyl sulfone) of sulfinic acid results from the reaction of sulfonyl cyanide and cyclopentadiene (for example, benzenesulfinic acid), and this requires a filtration step for the deposited solid products. Then, if deposition of the solid products is intended to be prevented completely, a great amount of water or hydrocarbon solvent has to be used, which increases the burden on waste water treatment and leaves a room for the improvement.

Further, in the method (iii) above, sulfonyl cyanide and cyclopentadiene are reacted under a pH condition of from 3 to 4 inclusive and it is difficult to completely prevent deposition of dimerization products (for example, benzenesulfinyl sulfone) of sulfinic acid (for example, benzenesulfinic acid) perhaps because a mixed solvent of water and a water-soluble solvent is used as a solvent. Moreover, the method (iii) gives a lower yield of ABH than the process (ii) described above.

Thus, an aim of the present invention is to overcome the above-mentioned problems caused when ABH is synthesized by the reaction of substituted sulfonyl cyanide and cyclopentadiene and provide a process for producing ABH safely and economically in a high purity and a high yield.

The inventors made various investigations to develop an improved process for producing ABH having a high purity smoothly and on an industrial scale. As a result, it has been found that by improving the methods (i) and (iV), that is, by mixing sulfonyl cyanide and cyclopentadiene continuously to prepare 3-sulfonyl-2-azabicyclo[2.2.1]hepta-2,5-diene as an intermediate, and continuously adding this reaction solution dropwise to water or a mixed solvent of water and a hydrocarbon solvent while keeping pH always within a range of from 4 to 7 inclusive by addition of an alkali to this reaction mixture, the time when the unstable 3-sulfonyl-2-azabicyclo[2.2.1]hepta-2,5-diene exists can be shortened to produce the aimed ABH in a high purity and a high yield, with an industrial safety and a good productivity. It has also been observed that even if the amount of the water and hydrocarbon solvent to be used is reduced in the case that the pH of the reaction mixture is controlled into a range of from 4 to 7 during the above-mentioned reaction, the reaction can proceed without precipitation of any solid dimerization products (for example, benzenesulfinyl sulfone) of sulfinic acid (for example, benzenesulfinic acid), and that in this case any problem such as impossibility of stirring during the reaction does not arise at all and the step of filtrating the solution can be omitted.

### SUMMARY OF THE INVENTION

The present invention relates to a process for producing a 2-azabicyclo[2.2.1]hept-5-en-3-one which comprises mixing a substituted sulfonyl cyanide represented by the following general formula (I):

R-SO₂CN (I)

wherein R represents an alkyl group or a phenyl group which may be substituted; with cyclopentadiene continuously, adding the resultant reaction solution continuously to water or a mixed solvent comprising water and a hydrocarbon solvent the resultant reaction mixture being maintained at a pH of from 4 to 7 inclusive.

### DETAILED DESCRIPTION OF THE INVENTION

In the substituted sulfonyl cyanide represented by the general formula (I) to be used in the present invention (hereinafter referred to as sulfonyl cyanide (I)), R represents an alkyl group or a phenyl group with or without a substituent. The alkyl group is preferably an alkyl group of from 1 to 4 carbon atoms, specifically, methyl group, ethyl group, n-propyl group, i-propyl group, n-butyl group, i-butyl group or t-butyl group. The phenyl group is preferably a non-substituted phenyl group or a substituted phenyl group represented by the following formula (II): 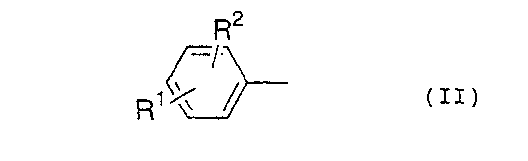 wherein R¹ and R² each independently represent a hydrogen atom, an alkyl group or a halogen atom.

In the formula (II), when R¹ and/or R² is an alkyl group, it is preferably an alkyl group of from 1 to 4 carbon atoms, and preferably, methyl group or ethyl group. Further, if R¹ and/or R² is a halogen atom in the formula (II), it is preferably a chlorine atom, bromine atom or fluorine atom.

Among them, the group R in the sulfonyl cyanide (I) is, more preferably methyl group, ethyl group, phenyl group, or p-tolyl group and, accordingly, in the present invention, methanesulfonyl cyanide, ethanesulfonyl cyanide, benezenesulfonyl cyanide, p-toluenesulfonyl cyanide or a mixture of two or more of them is used preferably as the sulfonyl cyanide (I), and benzenesulfonyl cyanide, p-toluenesulfonyl cyanide or a mixture thereof is more preferable.

The process for producing the sulfonyl cyanide (I) to be used in the present invention is not particularly limited, and it may be produced by any known method. In addition, the purity of the sulfonyl cyanide (I) to be used in the present invention is not particularly limited, but those having a purity of 70% or higher are generally used preferably since aimed ABH can be obtained smoothly.

The process for producing cyclopentadiene to be used in the present invention is not particularly limited, and it may be produced by any known method. In addition, there is no particular restriction on the purity of cyclopentadiene. Among them, use of cyclopentadiene formed by thermally decomposing dicyclopentadiene just after the preparation is preferred since the content of impurities is small, and post-treatment after production of ABH is easy.

In the process of the present invention, it is preferred to use cyclopentadiene at a ratio of one mol or more based on one mol of sulfonyl cyanide (I) and it is more preferred to use cyclopentadiene at a ratio of from 1 to 5 mol inclusive based on one mol of sulfonyl cyanide (I), particularly, in the view of economy and easiness of the post-treatment.

When mixing sulfonyl cyanide (I) and cyclopentadiene, use of a solvent is optional. Such a solvent is not particularly limited so far as it is inert to the cycloaddition reaction of sulfonyl cyanide (I) and cyclopentadiene. Examples thereof include aliphatic or aromatic hydrocarbons such as pentane, hexane, heptane, benzene, toluene, xylene; chlorinated hydrocarbons such as methylene chloride, chloroform and dichlorobenzene; ketones such as acetone, methyl isopropyl ketone and methyl isobutyl ketone; ethers such as diethyl ether, diisopropyl ether and methyl t-butyl ether. If these solvents are used, they may be used alone or as a mixture of two or more of them. In general, the weight of the solvent to be used is preferably from 0.1 to 20 parts by weight inclusive, and more preferably, from 0.1 to 5 parts by weight inclusive based on the one part by weight of sulfonyl cyanide (I) from the viewpoint of economy.

The temperature while mixing sulfonyl cyanide (I) and cyclopentadiene is preferably within a range from 0 to 50°C inclusive. From the viewpoint of stability of 3-sulfonyl-2-azabicyclo[2.2.1]hepta-2,5-diene, which is an intermediate, the temperature is more preferably within a range from 0 to 30°C inclusive.

The method for mixing sulfonyl cyanide (I) and cyclopentadiene may be a method of supplying sulfonyl cyanide (I) and cyclopentadiene simultaneously into a mixing apparatus. If solvent is used together, the solvent may be in the state that the solvent is being blended with only one or both of sulfonyl cyanide (I) and cyclopentadiene. The mixing apparatus may be any known apparatus such as a stirring type reaction vessel or a tube type reaction vessel. In order to add the reaction solution obtained by the mixing sulfonyl cyanide (I) and cyclopentadiene continuously to water or a mixed solvent of water and a hydrocarbon solvent, the mixing apparatus is connected to a reaction vessel wherein water or the mixed solvent of water and the hydrocarbon solvent is charged, in the manner that the prepared reaction solution can be continuously sent to the reaction vessel.

The residence time for the mixing sulfonyl cyanide (I) and cyclopentadiene may vary depending on the temperature upon the mixing or, if a solvent is used together, the kind of the solvent. The residence time is usually within a range from 5 to 600 minutes inclusive, preferably within a range from 5 to 120 minutes inclusive, and more preferably, within a range from 10 to 90 minutes inclusive from the viewpoint of the yield of aimed product, ABH. If the residence time is less than 5 minutes, the cycloaddition reaction of sulfonyl cyanide (I) and cyclopentadiene trends to proceed insufficiently, so that the yield of ABH decreases. If the residence time is exceeds 600 minutes, 3-sulfonyl-2-azabicyclo[2.2.1]hepta-2,5-diene, which is an intermediate produced by the cycloaddition reaction, trends to decompose so that the yield of ABH decreases.

Subsequently, the thus prepared reaction solution of sulfonyl cyanide (I) and cyclopentadiene is continuously added to water or a mixed solvent of water or a hydrocarbon solvent so as to be subjected to hydrolysis reaction.

The ratio of water to be used is usually within a range from 1 to 200 parts by mole, and preferably within a range from 1 to 50 parts by mole based on one mole of the sulfonyl cyanide (I), in view of easiness of the post-treatment.

Examples of the hydrocarbon solvent include aliphatic or aromatic hydrocarbons such as pentane, hexane, heptane, benzene, toluene and xylene. These may be used alone or as a mixture of two or more. When the hydrocarbon solvent is used, the weight thereof is preferably within a range from 0.005 to 200 parts by weight, and more preferably, within a range from 0.01 to 50 parts by weight based on one part by weight of water from the viewpoint of economy.

In the present invention, it is important that when adding the reaction solution of the sulfonyl cyanide (I) and cyclopentadiene continuously to water or a mixed solvent of water and a hydrocarbon solvent, the pH of the reaction mixture is constantly observed in the manner that the pH of the reaction mixture is kept within a range of from 4 to 7 inclusive. If the pH of the reaction mixture is less than 4, solid products such as dimerization products (for example, benzenesulfinyl sulfone) of sulfinic acid (for example, benzenesulfinic acid) are deposited in the reaction mixture to make stirring difficult upon the reaction, and a filtration step for separating the solid products is required which makes the reaction step complicated and lowers the yield and the purity of ABH. On the other hand, if the pH of the reaction mixture exceeds 7, hydrolysis of the resultant ABH proceeds to lower the yield. In the present invention, it is preferred to keep the pH of the reaction mixture within a range of from 4 to 6.5 inclusive, more preferably from pH of 4.2 to 5.5 inclusive for preventing deposition of the solid materials, and preventing hydrolysis of the resultant ABH.

As a method of keeping the pH of the reaction mixture within a range of from 4 to 7 inclusive, there is preferably adopted a method of optionally adding one or more of organic and inorganic alkali compounds not hindering the reaction to the reaction mixture while always observing the pH of the reaction mixture. In particular, it is preferred to keep the pH within a range of from 4 to 7 inclusive by adding an aqueous solution of one or more inorganic alkali compounds such as alkali metal hydroxides (for example, sodium hydroxide and potassium hydroxide), alkaline earth metal hydroxides (for example, calcium hydroxide, magnesium hydroxide and barium hydroxide), alkali metal carbonates (for example, sodium carbonate and potassium carbonate), alkaline earth metal carbonates (for example, calcium carbonate, magnesium carbonate and barium carbonate), alkali metal bicarbonates (for example, sodium hydrogen carbonate and potassium hydrogen carbonate) to the reaction mixture since the pH can be controlled easily.

The hydrolysis reaction is conducted preferably within a range of from 0 to 50°C inclusive, and more preferably, within a range of from 5 to 30°C inclusive in view of stability of the produced ABH in the reaction mixture.

The hydrolysis reaction is preferably conducted under stirring, so that the reaction can proceed smoothly. The reaction time can be controlled depending on the kind of sulfonyl cyanide (I), the amounts and the ratio of sulfonyl cyanide (I) and cyclopentadiene to be used, the amount of the hydrocarbon solvent and water to be used, the reaction temperature, the scale of the reactor, and the like. It is generally preferred to conduct the hydrolysis reaction for a time within a range from 30 minutes to 48 hours including the addition time (dropping time) of the reaction solution of the sulfonyl cyanide (I) and cyclopentadiene. The reaction may be conducted in a batch manner or a continuous manner.

As described above, the hydrolysis reaction is conducted while keeping the pH of the reaction mixture within a range of from 4 to 7, so as to produce the aimed ABH. Thereafter, the reaction can be terminated by controlling the pH of the reaction mixture which exceeds 7 and is 8 or lower.

The reaction mixture which contains ABH obtained as described above can be used without isolating ABH from the reaction mixture, as a starting material for synthesizing carbocyclic nucleoside or the like, but it is preferred to isolate ABH from the reaction mixture.

The method for isolating ABH from the reaction mixture is not particularly limited, and any method can be used so long as it is a method capable of isolating ABH smoothly. As the method for isolating ABH from the reaction mixture, preferred is a method of: extracting the reaction mixture with an appropriate extraction solvent in the case that only water is used, or separating the reaction mixture into an aqueous layer and a hydrocarbon solvent layer, recovering the aqueous layer and extracting ABH contained in the aqueous layer by an appropriate extraction solvent in the case that the mixed solvent of water and the hydrocarbon solvent; and then distilling off the extraction solvent to give the aimed ABH, by which the ABH can be obtained in a high yield and a high purity. As the extraction solvent in this case, any solvent can be used so long as it is a solvent capable of extracting ABH from the aqueous layer, but a chlorinated hydrocarbon solvent such as methylene chloride, chloroform or dichloroethane is preferably used.

In the isolating method as described above, if the extraction treatment by the extraction solvent is conducted after passing the aqueous layer containing ABH through an activated carbon packed column or charging activated carbon in the aqueous layer to remove impurities such as oily products derived from cyclopentadiene contained in the aqueous layer, before the extraction treatment of the aqueous layer by the extraction solvent, ABH in a higher purity can be obtained. Among them, the method of passing the aqueous layer containing ABH through the activated carbon packed column is extremely effective for removing impurities. The kind of the activated carbon to be used for the removal of the impurities is not particularly limited, but any activated carbon can be used and, among them, KURARAY COAL GC-F (manufactured by Kuraray Chemical Co., Ltd.) is preferably used. The shape, the structure and the size of the activated carbon packed column are not particularly limited, and can be determined depending on situations.

ABH obtained as described above, has a sufficiently high purity as such, and can be used effectively as a material for synthesizing carbocyclic nucleoside or the like, but it can be further purified or made easily handlable by optionally conducting distillation, activated carbon-treatment, sublimation or recrystallization further.

In the isolating step described above, sulfinate [R-SO₂M (M represents a salt-forming cation] remains in the aqueous layer after isolating ABH by conducting extraction treatment, for example, by using the chlorinated hydrocarbon solvent. If cyanogen chloride is added to the aqueous layer containing sulfinate after the extraction treatment, since sulfinate is easily converted to sulfonyl cyanide (I), the sulfonyl cyanide (I) produced by the conversion may be reutilized as a starting material for producing ABH.

According to the present invention, 2-azabicyclo[2.2.1]hept-5-en-3-one can be safely and economically produced in a high purity and a high yield. Since the amounts of water and the hydrocarbon solvent to be used can be reduced, the burden on treating waste water is remarkably mitigated.

### EXAMPLES

The present invention will be described more specifically by way of examples, but the present invention is not restricted to them.

### Reference Example 1 <Synthesis of benzenesulfonyl cyanide>

In a four-necked flask (200 ml volume), 70 g of water, 2 g of methylene chloride and 32.0 g (0.16 mol) of sodium benzenesulfinate dihydrate were charged, and then cooled to 3°C. Then, 10.7 g (0.17 mol) of gaseous cyanogen chloride was introduced thereto for about 15 minutes while keeping the internal temperature at 3 to 6°C inclusive. After introducing cyanogen chloride, the reaction mixture was stirred at 5°C for 30 minutes and then the mixture was transferred into a separation funnel. The organic layer was separated, and 5 g of methylene chloride was added to the aqueous layer to apply extraction treatment and, the extract and the organic layer were combined, dried over magnesium sulfate, and then methylene chloride was distilled off under reduced pressure to obtain 24.8 g (0.15 mol) of benezenesulfonyl cyanide (yield: 93.8%).

### Reference Example 2 <Synthesis of p-toluenesulfonyl cyanide>

The same procedures as those in Reference Example 1 were conducted except for using 34.3 g (0.16 mol) of sodium p-toluenesulfinate hydrate instead of 32.0 g (0.16 mol) of sodium benzenesulfinate dihydrate to obtain 27.5 g (0.15 mol) of p-toluenesulfonyl cyanide (yield: 93.8%).

### Reference Example 3 <Production of cyclopentadiene>

Into a four-necked flask (500 ml volume) equipped with a simple distillation device, 300 g (2.27 mol) of dicyclopentadiene was charged. Dicyclopentadiene was thermally decomposed and distilled while keeping the internal temperature at 155 to 160°C inclusive and the distilling temperature at 50 to 55°C inclusive to obtain 185 g (2.80 mol) of cyclopentadiene (yield: 61.7%).

### Example 1

A large portion of a Teflon tube (inner diameter: 4 mm, length: 2450 mm, and inner volume: 30.8 ml) to which two liquid-feeding pumps were in parallel connected through a "Y"-shaped joint was immersed into a water bath so that the liquid temperature in the tube could be controlled. The tip of the tube was connected to a four-necked flask (500 ml volume) equipped with a dropping funnel, a pH meter and a thermometer. Into this four-necked flask 130 g of water and 10 g of toluene were charged, and then the flask was cooled to 10°C or lower. Then, by using two liquid-feeding pumps simultaneously, 164 ml of a solution wherein 103. 2 g (0.58 mol) of benzenesulfonyl cyanide obtained in Reference Example 1 were dissolved in 100 g of methylene chloride at a rate of 0.91 ml/min and 54.0 g (0.80 mol, purity: 99.5%) of cyclopentadiene obtained in Reference Example 3 at a rate of 0.37 ml/min were feeded simultaneously in the Teflon tube, and the reaction solution of benzenesulfonyl cyanide and cyclopentadiene mixed in the Teflon tube was added dropwise continuously to the mixed solvent of water and toluene in the flask while stirring. At this time, the residence time of the reaction solution of benzenesulfonyl cyanide and cyclopentadiene mixed inside the Teflon tube was 23 minutes, and the temperature of the water bath to which the Teflon tube was immersed was kept 10°C or lower. During the continuous addition of the reaction solution of benzenesulfonyl cyanide and cyclopentadiene mixed inside the Teflon tube to the flask, the pH of the reaction mixture was continuously measured simultaneously, and an aqueous solution of 25% sodium hydroxide was added dropwise to the flask to keep the pH of the reaction mixture within a range from 4.4 to 4.6 inclusive. After the feeding, the temperature of the reaction mixture was kept 10°C and further the reaction mixture was stirred for 30 minutes. Then, an aqueous solution of 25% sodium hydroxide was added dropwise thereto to adjust the pH of the reaction mixture to 7.5. A portion of this reaction mixture was sampled and subjected to internal standard analysis by HPLC (high performance liquid chromatography) [column: Inertsil ODS-2 (inner diameter: 4.6 mm, length: 150 mm), eluent: 2 / 8 = MeOH/1 mM-KH₂PO₄ (adjusted to pH = 3.2, using 1M-H₃PO₄), flow rate: 1 ml/min, and detection wavelength: UV 225 nm] to find that 60.0 g (0.55 mol) of 2-azabicyclo[2.2.1]hept-5-en-3-one was contained in the reaction mixture (yield: 95.0%).

### Example- 2

The same procedures as in Example 1 were conducted except that a solution wherein 103.2 g (0.58 mol) of benzenesulfonyl cyanide were dissolved in 100 g of o-dichlorobenzene was used instead of the solution wherein 103. g (0.58 mol) of benzenesulfonyl cyanide were dissolved in 100 g of methylene chloride. The resultant reaction mixture was analyzed by HPLC in the same manner of Example 1 to find that 59.7 g (0.55 mol) of 2-azabicyclo[2.2.1]hept-5-en-3-one was obtained (yield: 94.3%).

### Example 3

The same procedures as in Example 1 were conducted except that a solution wherein 105.1 g (0.58 mol) of p-toluenesulfonyl cyanide were dissolved in 100 g of o-dichlorobenzene was used instead of the solution wherein 103. 2 g (0.58 mol) of benzenesulfonyl cyanide were dissolved in 100 g of methylene chloride. The resultant reaction mixture was analyzed by HPLC in the same manner of Example 1 to find that 59.5 g (0.55 mol) of 2-azabicyclo[2.2.1]hept-5-en-3-one was obtained (yield: 94.0%).

### Comparative Example 1

In a four-necked flask (500 ml volume) equipped with a nitrogen flowing tube and a thermometer, 54.0 g (0.80 mol) of 98.5% cyclopentadiene, 10 g of toluene and 130 g of water were charged, and then the inner temperature thereof was controlled to 10°C. Then 103.2 g (0.58 mol) of 94.3% benzenesulfonyl cyanide was added dropwise thereto from a dropping funnel for about 3 hours while keeping the inner temperature of the flask in a range of from 8 to 15°C inclusive. During the addition, the pH of the reaction mixture in the flask was continuously measured, and an aqueous solution of 25% sodium hydroxide was added dropwise thereto to keep the pH of the reaction mixture within a range from 4.4 to 4.7 inclusive. After the addition, the reaction mixture was further stirred at the same temperature for 30 minutes, and an aqueous solution of 25% sodium hydroxide was added dropwise thereto to increase the pH of the reaction mixture to 7.5, and the reaction mixture was transferred into a separation funnel to separate the aqueous layer. A portion of this aqueous layer was sampled and subjected to internal standard analysis by HPLC in the same manner of Example 1 to find that 52.4 g (0.48 mol) of 2-azabicyclo[2.2.1]hept-5-en-3-one was contained in the layer (yield: 82.8%).

## Claims

1. A process for producing 2-azabicyclo[2.2.1]hept-5-en-3-one which comprises mixing a substituted sulfonyl cyanide represented by the following general formula (I):
R-SO₂CN (I)
wherein R represents an alkyl group or a phenyl group which may be substituted;
with cyclopentadiene continuously, and adding the resultant reaction solution continuously to water or a mixed solvent comprising water and a hydrocarbon solvent, the resultant reaction mixture being maintained at a pH of from 4 to 7 inclusive.

2. The process of claim 1, wherein R is an alkyl group selected from the group consisting of methyl group, ethyl group, n-propyl group, i-propyl group, n-butyl group, i-butyl group and t-butyl group.

3. The process of claim 1, wherein R is a phenyl group selected from the group consisting of a nonsubstituted phenyl group and a substituted phenyl group represented by the following formula (II): 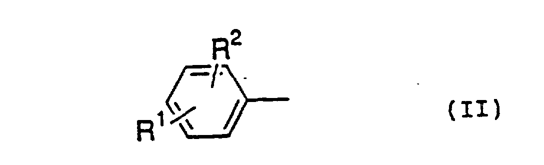 wherein R¹ and R² each independently represent a hydrogen atom, an alkyl group or a halogen atom.

4. The process according to any preceding claim, wherein the residence time for the continuous mixing of the substituted sulfonyl cyanide represented by the general formula (I) with cyclopentadiene is within a range from 5 to 600 minutes inclusive.

5. The process according to claim 4, wherein the residence time is within a range from 5 to 120 minutes inclusive.

6. The process according to claim 4, wherein the residence time is within a range from 10 to 90 minutes inclusive.

7. The process according to any preceding claim, wherein the temperature while continuously mixing the substituted sulfonyl cyanide represented by the general formula (I) with cyclopentadiene is within a range from 0 to 50°C inclusive.

8. The process according to any preceding claim, wherein the hydrocarbon solvent is selected from the group consisting of an aliphatic hydrocarbon, an aromatic hydrocarbon and mixed solvent thereof.

9. The process according to any preceding claim, wherein the pH ranges from 4 to 6.5 inclusive.

## Patentansprüche

1. Verfahren zur Herstellung von 2-Azabicyclo[2.2.1]hept-5-en-3-on, umfassend kontinuierliches Vermischen eines durch die nachstehende allgemeine Formel (l) wiedergegebenen substituierten Sulfonylcyanids:
R-SO₂CN (I)
worin R eine Alkylgruppe oder eine Phenylgruppe, die substituiert sein kann, wiedergibt;
mit Cyclopentadien und kontinuierliche Zugabe der erhaltenen Reaktionslösung zu Wasser oder einem gemischten Lösungsmittel, umfassend Wasser und ein Kohlenwasserstofflösungsmittel, wobei das erhaltene Reaktionsgemisch bei einem pH-Wert von 4 bis 7, einschließlich, gehalten wird.

2. Verfahren nach Anspruch 1, wobei R eine aus der Gruppe, bestehend aus Methylgruppe, Ethylgruppe, n-Propylgruppe, i-Propylgruppe, n-Butylgruppe, i-Butylgruppe und t-Butylgruppe, ausgewählte Alkylgruppe ist.

3. Verfahren nach Anspruch 1, wobei R eine Phenylgruppe darstellt, ausgewählt aus der Gruppe, bestehend aus einer nichtsubstituierten Phenylgruppe und einer durch die nachstehende Formel (II) wiedergegebenen substituierten Phenylgruppe: 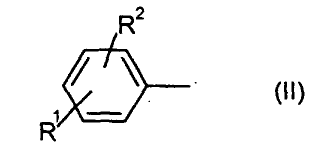 worin R¹ und R² jeweils unabhängig ein Wasserstoffatom, eine Alkylgruppe oder ein Halogenatom wiedergeben.

4. Verfahren nach einem vorangehenden Anspruch, wobei die Verweilzeit für das kontinuierliche Vermischen des durch die allgemeine Formel (l) wiedergegebenen substituierten Sulfonylcyanids mit Cyclopentadien im Bereich von 5 bis 600 Minuten, einschließlich, liegt.

5. Verfahren nach Anspruch 4, wobei die Verweilzeit im Bereich von 5 bis 120 Minuten, einschließlich, liegt.

6. Verfahren nach Anspruch 4, wobei die Verweilzeit im Bereich von 10 bis 90 Minuten, einschließlich, liegt.

7. Verfahren nach einem vorangehenden Anspruch, wobei die Temperatur während des kontinuierlichen Vermischens des durch die allgemeine Formel (l) wiedergegebenen substituierten Sulfonylcyanids mit Cyclopentadien im Bereich von 0 bis 50°C, einschließlich, liegt.

8. Verfahren nach einem vorangehenden Anspruch, wobei das Kohlenwasserstofflösungsmittel aus der Gruppe, bestehend aus einem aliphatischen Kohlenwasserstoff, einem aromatischen Kohlenwasserstoff und gemischtem Lösungsmittel davon, ausgewählt ist.

9. Verfahren nach einem vorangehenden Anspruch, wobei der pH-Wert im Bereich von 4 bis 6,5, einschließlich, liegt.

## Revendications

1. Procédé pour produire la 2-azabicyclo [2.2.1] hept-5-èn-3-one qui comprend l'opération consistant à mélanger un cyanure de sulfonyle substitué, représenté par la formule générale suivante (1) :
R-SO₂CN (I)
dans laquelle R représente un groupe alkyle ou un groupe phényle qui peut être substitué;
avec le cyclopentadiène en continu, et à ajouter la solution réactionnelle résultante en continu à l'eau ou à un solvant mixte comprenant de l'eau et un solvant hydrocarboné, le mélange réactionnel résultant étant maintenu à un pH allant de 4 à 7 inclus.

2. Procédé selon la revendication 1, dans lequel R est un groupe alkyle choisi dans le groupe consistant en un groupe méthyle, groupe éthyle, groupe n-propyle, groupe i-propyle, groupe n-butyle, groupe i-butyle et groupe t-butyle.

3. Procédé selon la revendication 1, dans lequel R est un groupe phényle choisi dans le groupe consistant en un groupe phényle non substitué et en un groupe phényle substitué représenté par la formule suivante (II) : 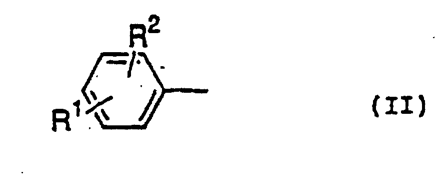 dans laquelle R¹ et R² représentent chacun indépendamment un atome d'hydrogène, un groupe alkyle ou un atome d'halogène.

4. Procédé selon l'une quelconque des revendications précédentes, dans lequel la durée de séjour pour le mélange continu du cyanure de sulfonyle substitué, représente par la formule générale (I), avec le cyclopentadiène se situe dans une plage allant de 5 à 600 minutes inclus.

5. Procédé selon la revendication 4, dans lequel la durée de séjour se situe dans une plage comprise allant de 5 à 120 minutes inclus.

6. Procédé selon la revendication 4, dans lequel la durée de séjour se situe dans une plage allant de 10 à 90 minutes inclus.

7. Procédé selon l'une quelconque des revendications précédentes, dans lequel tout en continuant à mélanger le cyanure de sulfonyle substitué représenté par la formule générale (1) avec le cyclopentadiène, la température se situe dans une plage allant de 0 à 50°C inclus.

8. Procédé selon l'une quelconque des revendications précédentes, dans lequel le solvant hydrocarboné est choisi dans le groupe consistant en un hydrocarbure aliphatique, un hydrocarbure aromatique et un solvant mixte de ceux-ci.

9. Procédé selon l'une quelconque des revendications précédentes, dans lequel le pH est compris dans une plage allant de 4 à 6,5 inclus.
